**Europäisches Patentamt**

**European Patent Office**

**Office européen des brevets**

(11) Veröffentlichungsnummer: **0 267 356**
**B1**

(12) **EUROPÄISCHE PATENTSCHRIFT**

(45) Veröffentlichungstag der Patentschrift: **23.01.91**

(21) Anmeldenummer: **87104558.9**

(22) Anmeldetag: **27.03.87**

(51) Int. Cl.⁵: **G 01 N 33/567,**
**G 01 N 33/68, G 01 N 33/543**

(54) Verfahren und Testelement zum Auffinden von Zelläsionen.

(30) Priorität: **10.11.86 LU 86654**

(43) Veröffentlichungstag der Anmeldung:
**18.05.88 Patentblatt 88/20**

(45) Bekanntmachung des Hinweises auf die
Patenterteilung:
**23.01.91 Patentblatt 91/04**

(84) Benannte Vertragsstaaten:
**AT BE CH DE FR GB IT LI NL SE**

(56) Entgegenhaltungen:
**EP-A-0 163 304**
**US-A-4 169 138**

**CHEMICAL ABSTRACTS, Band 101, Nr. 5, 30.
Juli 1984, Columbus, Ohio, USA; S.I. DANTO et
al.: "Immunocytochemical analysis of
intermediate filaments in embryonic heart cells
with monoclonal antibodies to desmin" Seite
395, Spalte 2, Zusammenfassung-Nr. 37046z**

(73) Patentinhaber: **PROGEN Biotechnik GmbH**
**Im Neuenheimer Feld 519**
**D-6900 Heidelberg (DE)**

(72) Erfinder: **Bruder, Gerda, Dr.**
**Bergheimer Strasse 110A**
**D-6900 Heidelberg (DE)**
Erfinder: **Franke, Werner Wilhelm, Prof.Dr.**
**Landfriedstrasse 6**
**D-6900 Heidelberg (DE)**

(74) Vertreter: **Hach, Hans Karl, Dr.**
**Tarunstrasse 23**
**D-6950 Mosbach-Waldstadt (DE)**

(56) Entgegenhaltungen:
**CHEMICAL ABSTRACTS, Band 96, Nr. 1, 4.
Januar 1982, Columbus, Ohio, USA; G.
GABBIANI et al.: "Immunochemical
identification of intermediatesized filaments in
human neoplastic cells. A diagnostic aid for the
surgical pathologist" Seite 286, Spalte 2,
Zusammenfassung-Nr. 3170y**

# EP 0 267 356 B1

**Beschreibung**

Die Erfindung betrifft ein Verfahren und ein Testelement zum Auffinden von Zelläsionen und mit Zelläsionen einhergehenden Krankheiten in lebenden Organismen, bei denen gewebstypische Proteine unlöslicher Strukturen dem Immunsystem zugänglich werden, beziehungsweise in Form ihrer Fragmente ins Serum gelangen und dort die Bildung von Antikörpern auslösen, deren Antigene gewebstypische Abschnitte dieser Proteine darstellen.

Die mit der Plasmamembran assoziierten Proteine cytoplasmatischer Plaque-Strukturen sind durch hohe Unlöslichkeit in biochemischen Puffern ausgezeichnet, hier kommen auch Zelltyp-spezifische Hauptproteine vor. Besonders gut untersucht ist die Zusammensetzung der Desmosomen-Plaques, deren Hauptproteine Desmoglein, Plakoglobin und die Desmoplakine I und II histodiagnostisch typisch für Epithelien und epitheliale Tumoren wie auch Meningen und Menigiome sind. Das Auftreten von Fragmenten dieser Hauptproteine in Körperflussigkeiten stellt einen geeigneten Indikator für Zelläsionen und destruktive Prozesse in epithelialen und meningialen Gewebetypen dar. Das gilt entsprechend bezogen auf die jeweils zugeordneten Gewebe für alle eingangs genannten Proteine. Es ist also wünschenswert, solche Proteine zu identifizieren.

Es ist aus EP-A-0163304 bekannt, mit spezifischen Antikörpern histodiagnostisch Intermediärfilamentproteine zu identifizieren. Intermediärfilamentproteine sind Bestandteile des Cytoskeletts und jeweils typisch für eine Gewebeklasse. Eine solche Identifizierung ermöglicht es, Intermediärfilamentproteine von Metastasen oder Gewebsläsionen dem Ursprungsgewebe beziehungsweise dem Primärtumor zuzuordnen.

Histologische Untersuchungen sind umständlich und in ihrer Anwendung beschränkt und führen nur zum Erfolg, wenn die betreffende Gewebeart in vitro identifizierbar oder für eine Untersuchung zugänglich ist.

Aufgabe der Erfindung ist es, ein Verfahren und ein Testelement der eingangs genannten Art so auszugestalten, daß es in der Praxis leicht und auch vielfältig anzuwenden ist und eine möglichst große Entdeckungschance bietet.

Die bei Zelläsionen in die Körperflüssigkeit freigesetzten gewebstypischen Proteine unlöslicher Strukturen verursachen die Produktion entsprechender Antikörper. Mit dem Nachweis solcher Antikörper sind die fraglichen Fragmente nachzuweisen und damit auch die zugehörigen Zelläsionen. Dies macht sich die Erfindung zur Lösung der genannten Aufgabe zunutze.

Die Erfindung ist dadurch gekennzeichnet, daß ein Testpräparat aus den betreffenden gereinigten Proteinen beziehungsweise Fragmenten davon, die das Antigen tragen, in vitro, mit den in Blut, Blutserum, Cerebrospinalliquor, Urin, Fruchtwasser, Punktat oder anderen Körperflüssigkeiten eventuell enthaltenen Antikörpern in Kontakt gebracht wird und daß die Qualität und Quantität der an diese Testsubstanzen gebundenen Antikörper bestimmt wird.

Die gewebstypischen Proteine unlöslicher Strukturen können Cytoskelettproteine sein, vorzugsweise sind es Intermediärfilamentproteine. Bei Intermediärfilamentproteinen enthalten die Fragmente die alpha-helikalen Mittelstücke oder Teile davon. Solche Fragmente werden alpha-helikale Mittelstück-Fragmente genannt. Alle alpha-helikalen Mittelstück-Fragmente, die hier gemeint sind, tragen das Antigen und entsprechen den obengenannten charakteristischen Abschnitten.

Die Erfindung macht sich dabei die folgenden Erkenntnisse aus der Zellbiologie zunutze:

Intermediärfilament(IF)-Proteine, die als Bestandteile des Cytoskeletts zu den unlöslichen Zellbestandteilen gehören, sind durch hohe Stabilität ausgezeichnete Proteine von großer Zelltypspezifität. Die Zuordnung ergibt sich aus der nachfolgenden Tabelle I.

TABELLE 1

| Gewebe | Intermediärfilamentprotein |
|---|---|
| Muskel— | Desmin |
| Neuronal— | Neurofilamentproteine (NF—L, NF—M, NF—H) |
| Mesenchymal— | Vimentin |
| Astrozyten | Gliafilament |
| Epithel— | Cytokeratine |

Zum Beispiel deutet Desmin oder ein identifizierbares Fragment des Desmins in Körperflüssigkeit auf zerstörte Muskelzellen hin.

Das Immunsystem reagiert mit der Generation von spezifischen Antikörpern gegen die alpha-helikalen Mittelstücke und betreibt damit die Elimination dieser Fragmente. Solche Antikörper werden vermehrt gebildet bei entsprechendem Bedarf. Das Vorhandensein von IgM-Antikörpern deutet auf eine kurzfristige immunologische Antwort, während das Vorhandensein von IgG-Antikörpern auf das Vorhandensein einer langfristigen immunologischen Antwort deutet. Welche Antikörper vorliegen, wird durch die nach der Erfindung vorgesehene Qualitätsbestimmung ermittelt.

Wenn also zum Beispiel Muskelgewebe pathologisch zerstört wird, dann wird Desminfragment freigesetzt und die Produktion von Antikörpern, deren Antigen das alpha-helikale Mittelstück des Desmin ist, wird angeregt.

Im Gegensatz zum bekannten histologischen Verfahren, bei dem nach verhältnismäßig großen, aussagekräftigen Intermediärfilamentproteinen gesucht wird, sucht das erfinderische Verfahren nach im Serum enthaltenen, entsprechend aussagekräftigen Antikörpern, für die die Entdeckungswahrscheinlichkeit erheblich größer ist. Das bietet auch eher die Möglichkeit, die Häufigkeit der Kontakte und die Schnelligkeit, mit der diese Kontakte gefunden werden, auszuwerten zu einer Aussage darüber, ob sich das Ergebnis noch im Rahmen des unpathologischen Durchschnitts bewegt oder ob ein pathologischer Herd da ist und von welcher Intensität er ist. Außerdem kann dieser Herd, wie auch bei der bekannten histologischen Diagnose eindeutig einem der fünf Gewebetypen zugeordnet werden, da der Antikörper über sein Antigen, das ihm als Testpräparat angeboten wird, einem der Gewebetypen eindeutig zuordbar ist.

Das alpha-helikale Mittelstück ist als Diagnoseobjekt für Intermediärfilamentproteine ausreichend, weil es dem zugehörigen Gewebetyp eindeutig zuordbar ist. Es ist als Diagnoseobjekt günstig, weil es gegenüber den langgestreckten Schwänzen der Intermediärfilamentproteine relativ schwer verdaulich ist und im Gegensatz zu den intakten Intermediärfilamentproteinen im Serum löslich ist. Durch diese besonderen Eigenschaften der alpha-helikalen Mittelstücke wird die Produktion der gesuchten Antikörper relativ stark angeregt und damit ein relativ starkes Diagnosesignal erzeugt.

Das erfinderische Verfahren ist in vitro durchführbar, indem die Messung mit einer aus dem Organismus extrahierten Körperflüssigkeit erfolgt.

Die stattgefundenen Antikörperkontakte kann man mit bekannten Verfahren messen. Dazu können beispielsweise die angelagerten Antikörper markiert werden mit einem entsprechend präparierten weiteren Antikörper. Es ist aber auch möglich, mit einem markierten weiteren Antikörper die noch unbesetzten Positionen des Testpräparates zu besetzen. Dann kann man aus der erkennbaren Besetzungsdichte der markierten Antikörper schließen, wie viele Positionen bereits von Antikörpern aus dem Serum besetzt waren. Die Markierung kann physikalisch, zum Beispiel radioaktiv, oder biochemisch, zum Beispiel durch das Enzym Peroxidase erfolgen.

Die Diagnose kann mit Testelementen erfolgen. Eine entsprechende einfache Ausgestaltung eines solchen Testelements ist dadurch gekennzeichnet, daß ein Teststreifen, eine Testsonde oder dergleichen Testelement mit dem Testpräparat ausgestattet ist und daß dieses Testpräparat einem Zell- beziehungsweise Gewebetyp zugeordnet ist, indem es nur alpha-helikale Mittelstücke eines Typs von Intermediärfilamentproteinen enthält.

Ein solches Testelement ist nur in Verbindung mit einem Gewebetyp geeignet.

Man kann aber auch mit ein und demselben Test mit Bezugnahme auf mehrere, möglicherweise alle fünf Gewebetypen, untersuchen. Eine geeignete Ausgestaltung eines Testelements ist dadurch gekennzeichnet, daß ein Teststreifen, eine Testsonde oder dergleichen Testelement mit mehreren verschiedenen Testpräparaten ausgestattet ist und daß jedes Testpräparat nur alpha-helikale Mittelstücke eines Typs von Intermediärfilamentproteinen enthält.

Die Erfindung wird nun anhand einiger Verfahrensbeispiele und der beigefügten Zeichnung näher erläutert.

Beispiel 1
Nachweis von Läsionen im Epithelgewebe (Differentialdiagnostik)
In einen Milliliter Serum von Patientenblut werden die vorhandenen Antikörper gegen Cytokeratine immunologisch durch einen Sandwich-ELISA bestimmt. Hierzu dient eine Mikrotiterplatte, die in 19 verschiedenen Feldern mit gereinigten alpha-helikalen Mittelstücken der 19 verschiedenen Cytokeratine (CK Nr. 1 bis 19) beschichtet ist. Die Portion der Körperflüssigkeit wird darüber geschüttet. Anschließend wird das Präparat sauber gewaschen.
Die spezifisch vom Präparat absorbierten Antikörper aus dem Patientenblut werden identifiziert immunologisch mit spezifischen Antiköpern gegen IgM oder IgG, die ihrerseits enzymatisch mit Peroxidase markiert sind. Zur Standardisierung wird gereinigtes IgG oder IgM aus menschlichem Blut verwendet.

Beispiel 2 und 3
Wie Beispiel 1, mit dem einzigen Unterschied, daß anstelle von Serum von Patientenblut eine andere Körperflüssigkeit eingesetzt wird, nämlich:
bei Beispiel 2 Urin und
bei Beispiel 3 Synovialpunktat.

Beispiel 4 bis 6
Wie Beispiel 1 bis 3 mit dem einzigen Unterschied, daß zur Standardisierung gentechnisch gewonnenes, reines menschliches IgM oder IgG eingesetzt wird.

## EP 0 267 356 B1

**Patentansprüche**

1. Verfahren zum Auffinden von Zelläsionen und mit Zelläsionen einhergehenden Krankheiten in lebenden Organismen, bei denen gewebstypische Proteine unlöslicher Strukturen dem Immunsystem zugänglich werden, beziehungsweise in Form ihrer Fragmente ins Serum gelangen und dort die Bildung von Antikörpern auslösen, deren Antigene gewebstypische Abschnitte dieser Proteine darstellen, dadurch gekennzeichnet, daß ein Testpräparat aus den betreffenden gereinigten Proteinen beziehungsweise Fragmenten davon, die das Antigen tragen, in vitro, mit den in Blut, Blutserum, Cerebrospinalliquor, Urin, Fruchtwasser, Punktat oder anderen Körperflüssigkeiten eventuell enthaltenen Antikörpern in Kontakt gebracht wird und daß die Qualität und Quantität der an diese Testsubstanzen gebundenen Antikörper bestimmt wird.

2. Verfahren nach Anspruch 1, dadurch gekennzeichnet, daß ein Testpräparat aus gereinigten Cytoskelettproteinen beziehungsweise Fragmenten davon eingesetzt wird.

3. Verfahren nach Anspruch 2, dadurch gekennzeichnet, daß die Cytoskelettproteine Intermediärfilamentproteine sind und daß die Fragmente deren alpha-helikale Mittelstücke oder Teile dieser Mittelstücke enthalten.

4. Testelement zur Ausübung des Verfahrens nach einem der vorhergehenden Ansprüche, dadurch gekennzeichnet, daß ein Teststreifen, eine Testsonde oder dergleichen Testelement mit dem Testpräparat ausgestattet ist und daß dieses Testpräparat einem Zell- beziehungsweise Gewebetyp zugeordnet ist, indem es nur alpha-helikale Mittelstücke eines Typs von Intermediärfilamentproteinen enthält.

5. Testelement nach Anspruch 4, dadurch gekennzeichnet, daß ein Teststreifen, eine Testsonde oder dergleichen Testelement mit mehreren verschiedenen Testpräparaten ausgestattet ist und daß jedes Testpräparat nur alpha-helikale Mittelstücke eines Typs von Intermediärfilamentproteinen enthält.

**Revendications**

1. Procédé pour détecter des lésions cellulaires et des maladies inhérentes à des lésions cellulaires, dans des organismes vivants dans lesquels des protéines spécifiques d'un tissu, de structures indissociables, deviennent accessibles au système immunitaire ou parviennent respectivement, sous forme de leurs fragments, dans le sérum dans lequel elles déclenchent la formation d'anticorps, dont les antigènes représentent des segments de ces protéines qui sont spécifiques d'un tissu, caractérisé par le fait qu'une préparation expérimentale à base des protéines purifiées considérées, respectivement de fragments de ces dernières qui portent l'antigène, est mise en contact *in vitro* avec les anticorps éventuellement renfermés par le sang, par le sérum sanguin, par la liqueur cérébro-spinale, par les urines, par le liquide amniotique, par du liquide de paracentèse ou par d'autres fluides corporels; et que la qualité et la quantité des anticorps, liés à ces substances d'expérimentation, sont déterminées.

2. Procédé selon la revendication 1, caractérisé par le fait qu'on utilise une préparation expérimentale à base de protéines purifiées du cytosquelette, respectivement de fragments de ces dernières.

3. Procédé selon la revendication 2, caractérisé par le fait que les protéines du cytosquelette sont des protéines fibrillaires intermédiaires; et que les fragments renferment les zones centrales en hélices alpha desdites protéines, ou bien des parties de ces zones centrales.

4. Moyen de test pour la mise en oeuvre du procédé selon l'une des revendications précédentes, caractérisé par le fait qu'une lamelle expérimentale, une sonde expérimentale ou moyen expérimental similaire est muni(e) de la préparation expérimentale; et que cette préparation expérimentale est associée à un type respectivement cellulaire ou tissulaire, du fait qu'elle renferme seulement des zones centrales en hélices alpha d'un type de protéines fibrillaires intermédiaires.

5. Moyen de test selon la revendication 4, caractérisé par le fait qu'une lamelle expérimentale, une sonde expérimentale ou moyen expérimental similaire est muni(e) de plusieurs préparations expérimentales différentes; et que chaque préparation expérimentale renferme seulement des zones centrales en hélices alpha d'un type de protéines fibrillaires intermédiaires.

**Claims**

1. Process for the detection of cell lesions and the illnesses associated therewith in living organisms, in which tissue-typical proteins of insoluble structures are accessible to the immune system, or in the form of their fragments pass into the serum and initiate there the formation of antibodies, whose antigens represent tissue-typical portions of said proteins, characterized in that a test preparation from the particular purified proteins or fragments thereof and which carry the antigen is contacted in vitro with the antibodies possibly contained in the blood, blood serum, cerebrospinal liquor, urine, amniotic fluid, punctate or other body fluids and that the quality and quantity of the antibodies bound on said test substances is determined.

2. Process according to claim 1, characterized in that use is made of a test preparation of purified cytoskeleton proteins or fragments thereof.

3. Process according to claim 2, characterized in that the cytoskeleton proteins are intermediate filament proteins and that the fragments contain their alpha-helical central portions or parts of the latter.

4. Test device for performing the process according to one of the preceding claims, characterized in

4

that a test strip, test probe or similar test device is provided with said test preparation and that the latter is associated with a cell or tissue type, in that it only contains alpha-helical central portions of one type of intermediate filament proteins.

5. Test device according to claim 4, characterized in that a test strip, a test probe or similar test device is provided with several different test preparations and that each test preparation only contains alpha-helical central portions of one type of intermediate filament proteins.